# EUROPEAN PATENT SPECIFICATION

(11) **EP 1 549 246 B1**
(45) Date of publication and mention of the grant of the patent: **02.05.2012**
(21) Application number: 03754460.8
(22) Date of filing: 05.09.2003
(51) Int. Cl.: A61F 2/02, A61K 9/50, A61K 38/14, A61K 31/7036, A61K 31/545, A61P 31/04, A61P 19/08

(54) **Antibiotic micropheres for treatment of infections and osteomyelitis**
Antibiotische Mikrokügelchen zur Behandlung von Infektionen und Osteomyelitis
Microspheres antibiotiques pour le traitement d'infections et de l'osteomyelite

(30) Priority: 05.09.2002 US 408496 P; 05.09.2002 US 408502 P
(43) Date of publication of application: 06.07.2005
(73) Proprietor: Ambrose, Catherine G., Houston, TX 77030 (US); Clyburn, Terry A., Houston, TX 77030 (US); Mikos, Antonios G., Houston, TX 77251 (US)
(72) Inventor: Ambrose, Catherine G., Houston, TX 77030 (US); Clyburn, Terry A., Houston, TX 77030 (US); Mikos, Antonios G., Houston, TX 77251 (US)
(74) Representative: Atkinson, Peter Birch
(86) International application number: PCT/US2003/028010
(87) International publication number: WO 2004/022000

(56) References cited:
- EP-A- 0 709 085
- WO-A-00/74650
- WO-A-02/26215
- WO-A-02/45689
- US-B1- 6 309 669
- US-B1- 6 461 631
- RAMCHANDANI, M. ET AL: "In vitro and in vivo release of ciprofloxacin from PLGA 50:50 implants" JOURNAL OF CONTROLLED RELEASE, vol. 54, no. 2, 1998, pages 167-175, XP002494334
- UENG , S.W. N ET AL: "In vivo study of hot compressing molded 50:50 poly (DL-lactide-co-glycolide) antibiotic beads in rabbits" JOURNAL OF ORTHOPAEDIC RESEARCH, vol. 20, no. 4, 20 February 2002 (2002-02-20), pages 654-661, XP002494335
- PRICE , J.S. ET AL: "Controlled release of antibiotics from coated orthopedic implants" JOURNAL OF BIOMEDICAL MATERIALS RESEARCH PART A, vol. 30, 1996, pages 281-286, XP002494336
- CHANGHONG, Y. ET AL: "Characterization and morphological analysis of protein-loaded poly(lactide-co-glycolide) microparticles prepared by water-in-oil-in-water emulsion technique" JOURNAL OF CONTROLLED RELEASE, vol. 32, no. 3, 1994, pages 231-241, XP002494337
- GARVIN, K.L. ET AL: "Polylactide/polyglycolide antibiotic implants in the treatment of osteomyelitis. A canine model" THE JOURNAL OF BONE AND JOINT SURGERY, vol. 76, no. 10, 1994, pages 1500-1506, XP002494338
- MARTINEZ B ET AL: "IN VITRO CIPROFLOXACIN RELEASE FROM POLY(LACTIDE-CO-GLYCOLIDE) MICROSPHERES" JOURNAL OF MICROENCAPSULATION, TAYLOR AND FRANCIS, BASINGSTOKE, GB, vol. 14, no. 2, 1 March 1997 (1997-03-01), pages 155-161, XP000643341 ISSN: 0265-2048
- LIN, S.-S. ET AL: "Development of a biodegradable antibiotic delivery system" CLINICAL ORTHOPAEDICS AND RELATED RESEARCH, 1999, pages 240-250, XP009105242
- KING , T.W. ET AL: "Development and in vitro characterization of vascular endothelial growth factor (VEGF)-loaded poly(DL-lactic-co-glycolic acid)/poly(ethylene glycol) microspheres using a solid encapsulation/single emulsion/solvent extraction technique" JOURNAL OF BIOMEDICAL MATERIALS RESEARCH, vol. 51, no. 3, 2000, pages 383-390, XP002494341

## Description

### TECHNICAL FIELD

This invention relates generally to microspheres capable of time releasing a drug and, more particularly to microspheres for implantation, injection, or other placement totally or partially within the body that are capable of near-linear controlled release of an antibiotic for an extended period of time for the treatment and prevention of infections involving the body.

### BACKGROUND ART

Historically, osteomyelitis treatment has consisted of debridement of infected tissues, irrigation with an antiseptic solution, and four to six weeks of parenteral antibiotic treatment. Due to poor penetration of the antibiotic into the infected bone site, high serum concentrations of the antibiotic need to be employed for extended periods of time in order to produce bactericidal levels within the bone tissue. These high serum levels can be associated with nephrotoxicity or ototoxicity, and can cause gastroinstestinal side effects. Due to the morbitiy associated with high serum levels of antibiotics, many local delivery methods have been described including bone cement with antibiotics, collagen sponge with gentamycin, polymeric carriers with various antibiotics, and calcium sulfate carriers of antibiotics.

The need for a local drug delivery system to deliver antibiotics directly to the infection site led many physicians to mix antibiotics and polymethyl methacrylate (PMMA) bone cement into beads and place these beads into the debrided bone defect. Typically, these beads have been shown to deliver non-linear doses of antibiotic over the course of only a few weeks, and after the antibiotic has been eluted, the cement beads must be removed, as the cement is not biodegradable and may become a nidus for infection. Relevant prior art includes US6410056.

Infection may complicate any surgical treatment. Areas of high risk include fractures of bone treated with metal rods, plates or external fixators. The risk is particularly high if the fracture was open (compound fractures). Other surgical procedures are also at risk including vascular bypass surgery with the use of artificial graft material, general surgical procedures such as hernia repair and various procedures performed about the uterus and bladder. Once established, these infections are typically treated with surgical drainage and systemic antibiotics. Just as in the treatment of osteomyelitis, the treatment for infection may be prolonged, costly and may fail. There exists a need for a safe, effective local antibiotic delivery formulation that will improve healing and prevent complications.

The present invention is distinguished over the prior art in general, and these patents in particular by biodegradable microspheres implanted, injected, or otherwise placed totally or partially within the body that are capable of near-linear controlled release of an antibiotic for a predetermined period of time for the treatment and prevention of infections involving the body. The microspheres are formed of polylactic-co-glycolic acid (PLGA) and an effective amount of antibiotic sufficient to produce bactericidal levels in body tissues, and may or may not include polyethylene glycol (PEG). The microspheres exhibit near-linear delivery of the antibiotic for at least 4 weeks at levels exceeding the minimum inhibitory concentration (MIC) for organisms commonly found to be the cause of infections. The microspheres allow antibiotics to be delivered at the time of various surgical treatments to decrease the occurrence of infection, and may be used for the treatment of open fractures, open reduction and internal fixation with metallic fixation of fractures, placement of joint replacement devices, and placement of various graft materials used in cardiovascular, general, gynecologic, and neurosurgical procedures.

### DISCLOSURE OF THE INVENTION

It is therefore an object of the present invention to provide antibiotic microspheres for the treatment and prevention of infections that are capable of near-linear release of the antibiotic for an extended period of time, and at levels exceeding the minimum inhibitory concentration (MIC) for organisms commonly found to be the cause of infection.

It is another object of this invention to provide a biodegradable microsphere antibiotic delivery system for the treatment and prevention of infections and osteomyelitis that eliminates the need for an additional surgery to remove the drug carrier.

Another object of this invention is to provide antibiotic microspheres for the treatment and prevention of infections and osteomyelitis that may remain at the site of implantation and do not inhibit tissue regeneration.

Another object of this invention is to provide antibiotic microspheres for the treatment and prevention of infections that deliver antibiotics at the time of various surgical treatments to decrease the occurrence of infection.

A further object of this invention is to provide antibiotic microspheres for the treatment and prevention of infections that can be easily and quickly implanted, injected, or otherwise placed totally or partially within the body at a site of actual or potential infection.

A still further object of this invention is to provide antibiotic microspheres for the treatment and prevention of infections that can be placed at a site of at a site of placement of metal rods, plates or metallic fixators, of joint replacement devices, and of graft materials used in cardiovascular, general, gynecologic, and neurosurgical procedures.

Other objects of the invention will become apparent from time to time throughout the specification and claims as hereinafter related.

The above noted objects and other objects of the invention are accomplished by the present biodegradable microspheres that are implanted, injected, or otherwise placed totally or partially within the body and are capable of near-linear controlled release of an antibiotic for a predetermined period of time for the treatment and prevention of infections involving the body. The microspheres are formed of polylactic-co-glycolic acid (PLGA) and an effective amount of antibiotic sufficient to produce bactericidal levels in body tissues, and may or may not include polyethylene glycol (PEG). The microspheres exhibit near-linear delivery of the antibiotic for at least 4 weeks at levels exceeding the minimum inhibitory concentration (MIC) for organisms commonly found to be the cause of infections. The microspheres allow antibiotics to be delivered at the time of various surgical treatments to decrease the occurrence of infection, and may be used for the treatment of open fractures, open reduction and internal fixation with metallic fixation of fractures, placement of joint replacement devices, and placement of various graft materials used in cardiovascular, general, gynecologic, and neurosurgical procedures.

### BRIEF DESCRIPTION OF THE DRAWINGS

Fig. 1 is a graph illustrating the *in-vitro* elution of the various microsphere formulations.
Fig. 2 is a graph illustrating the results of a repeatability study of the *in vitro* elution rates where two of the formulations were manufactured more than one year apart.
Fig. 3 is a graph illustrating the *in-vivo* tobramycin concentrations in tissue over time for two of the formulations tested.
Fig. 4 is a graph illustrating the percentage of animals testing positive for osteomyelitis in a study of rabbits in groups treated with various antibiotic microsphere formulations.
Fig. 5 is a graph illustrating the results of radiographic and histological grading of the bone specimens taken from the rabbit study.
Fig. 6 is a graph illustrating the concentration of tobramycin in the bones for the groups treated locally with tobramycin.
Fig. 7 is a graph illustrating the entrapment efficiency and elution rate over time of various microsphere formulations utilizing vancomycin.

### BEST MODE FOR CARRYING OUT THE INVENTION

The microsphere containing the antibiotic substance according to the present invention can be made of varying amounts of polylactic-co-glycolic acid (PLGA) with or without polyethylene glycol (PEG), and an effective cephalosporin antibiotic, using a water-in-oil-in-water (W/O/W), double-emulsion-solvent-extraction technique. In a preferred embodiment, the biodegradable microspheres are formed of from about 85% to about 99% by weight of polylactic-co-glycolic acid (PLGA) in a ratio of 50% lactic to 50% glycolic acid, from about 0% to about 5% by weight of polyethylene glycol (PEG); and an effective amount of an antibiotic agent sufficient to produce bactericidal levels in body tissues. The microspheres are characterized in that they exhibit near-linear delivery of the antibiotic agent for at least 4 weeks at levels exceeding the minimum inhibitory concentration (MIC) for organisms commonly found to be the cause of infections. The present invention will be more clearly understood with reference to the following examples.

### EXAMPLE 1- PLGA/tobramycin Drug Delivery System

### Preparation of Microspheres

In the following examples the polylactic-co-glycolic acid (PLGA) used was a high molecular weight blend of 50% lactic to 50% glycolic acid (Medisorb®), from Alkermes, Cincinnati, OH. Polyethylene glycol (PEG) and polyvinyl alcohol (PVA) were purchased from Sigma Aldrich, of St. Louis, MO. Tobramycin (Nebcin®), from Eli Lilly, Indianapolis, IN. was purchased in powder form, and all remaining chemicals were purchased from Fisher Scientific (Pittsburgh, PA).

Microparticles were prepared in many blends of PLGA/PEG/tobramycin using an established water-in-oil-in-water (W/O/W), double-emulsion-solvent-extraction technique. The size distribution of the microparticles was measured with a Coulter counter multisizer (model 0646, Coulter Electronics, Hialeah, FL) after suspending the particles in an Isoton II solution (Coutler Electronics).

The entrapment efficiency of the formulation was determined in duplicate by normalizing the amount actually entrapped to the starting amount, using the established solvent-extraction technique. 10mg of microparticles was dissolved in 1ml of dichloromethane for 6 hours at room temperature. The tobramycin was then extracted from the organic phase to the aqueous phase by mixing 1ml PBS and removing the aqueous portion. This was repeated every six hours for twenty-four hours and all aqueous aliquots tested for tobramycin concentration.

All tobramycin concentrations were performed using fluorescence polarization immunoassay (Abbot TDx System). Sensitivity of the tobramycin assay is defined as the lowest measurable concentration which can be distinguished from zero with 95% confidence and was determined to be 0.18 microgram per milliliter.

### In-Vitro Elution Rate Determination

By dry weight, the percentage of PEG in the formulations was either 0% or 5%, and the percentage of tobramycin was either 1%, 5%, or 10%. In all, six different formulations were studied for tobramycin elution rates. 25mg amounts of microparticles were measured and placed into 2ml glass vials containing 1ml PBS. Each microparticle formulation was tested in triplicate and placed in a water bath at 37° C. After 24 hours, the vials were centrifuged and the supernatant removed for tobramycin assay. 1ml of PBS was added to the vials and the vial replaced in the water bath. This was repeated once daily for one week, and then every second day for three additional weeks.

### In-Vivo Drug Release Characteristics

Two formulations were studied in a mouse muscle pouch model, the 10% tobramycin with either 0% or 5% PEG. 60 adult female ICR mice, weighing 20-24g were used for this investigation. Each animal was anesthetized using ketamine (150mg/kg) and xylazine (6mg/kg) IP injection. A small incision was made over the right quadriceps muscle and a small pouch was made in the muscle by blunt dissection. In thirty mice, 5mg of microspheres containing 10% tobramycin and 0% PEG were implanted into the pouch; in the remaining thirty mice, microspheres containing 10% tobramycin and 5% PEG were implanted. Each pouch was closed with a nonabsorbable suture to mark the location. The skin was closed with resorbable suture. All animals ambulated normally throughout the study, and no signs of local inflammation (swelling, tenderness) were visible.

For each of the two microsphere formulations tested, the mice were divided into 5 groups of six mice each and sacrificed sequentially at one day, four days, seven days, twenty-two days, and either 33 or 40 days post-surgery. At sacrifice, the scarred incision was reopened and the pouch located by the suture. Approximately 0.1g of tissue surrounding the suture was removed. Half of the tissue was placed in formalin for subsequent histological evaluation. The remaining half of the tissue was weighed and placed in 0.5ml PBS and macerated. The tissues from three mice in each group were randomly pooled together in each vial such that there were two vials for each timepoint for each group. The tissue was incubated for 2 hours at 37° C. After incubation, the vial was centrifuged and the supernatant filtered for tobramycin analysis. Tobramycin concentration is presented as amount of tobramycin per weight of muscle tissue.

The preserved tissue was cut into 5µm sections and stained with an H&E stain. Each slide was graded for inflammation by a blinded pathologist according to the following scale: 1 for no or minimal inflammation, 2 for moderate inflammation, and 3 for marked or severe inflammation.

### In Vitro Results

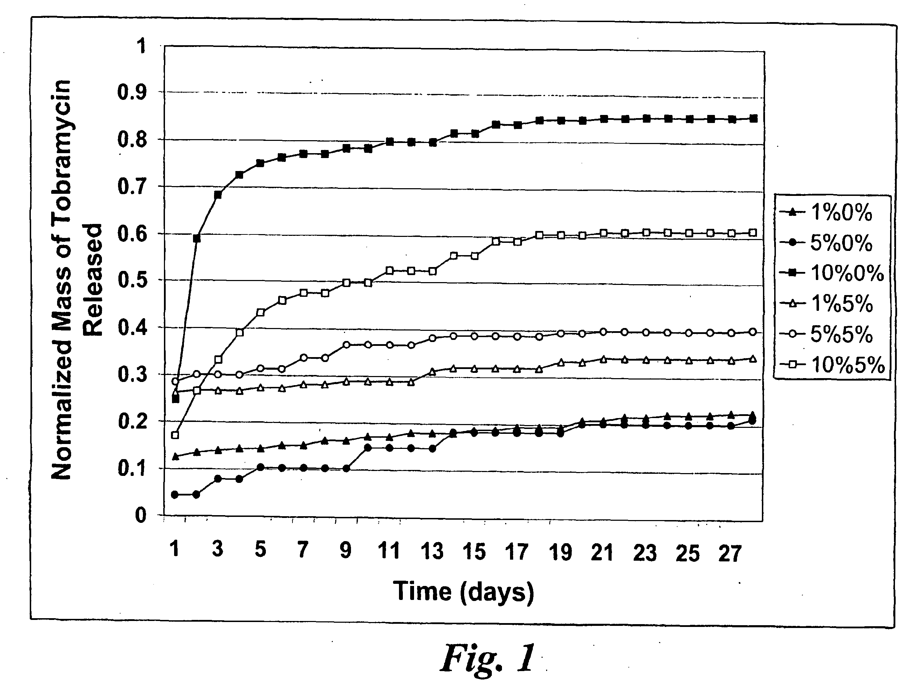

The *in-vitro* elution of the 6 microsphere formulations is shown in Fig. 1. In this figure, the amount of drug released has been normalized to the total amount present in the implanted microspheres. The entrapment efficiency for each formulation of microsphere ranged between 40.24% to 61.8%, as shown in Table 1 below. In general, adding PEG increased the entrapment efficiency. All microspheres were found to be on average 20 ± 1.6 µm in diameter.

Each formulation had a large initial release of tobramycin in the first 24 hours, followed by a few days of lowered release and then a few weeks of nearly steady release. Linear fits of the elution curves during the 7-28 day time period demonstrated correlations ranging from r²=0.7748 to 0.9770, indicating that the release of antibiotic is very linear over this time period. Table 1 shows the calculated average linear release of tobramycin for each formulation for days 7 through 28 in absolute amounts and percentage of total amount of drug.

**Table 1. Microsphere Characteristics and in vitro Elution**

| Microsphere Formulation | | | Entrapment | Average Release | |
|---|---|---|---|---|---|
| %PLGA | %tobramycin | %PEG | Efficiency | µg/day | %/day |
| 99 | 1 | 0 | 42.8% | 0.3852 | 0.48 |
| 95 | 5 | 0 | | 2.3586 | 0.37 |
| 90 | 10 | 0 | 45.8% | 4.4510 | 0.41 |
| 94 | 1 | 5 | 61.8% | 0.5131 | 0.33 |
| 90 | 5 | 5 | 40.2% | 1.3415 | 0.27 |
| 85 | 10 | 5 | 52.4% | 8.7916 | 0.67 |

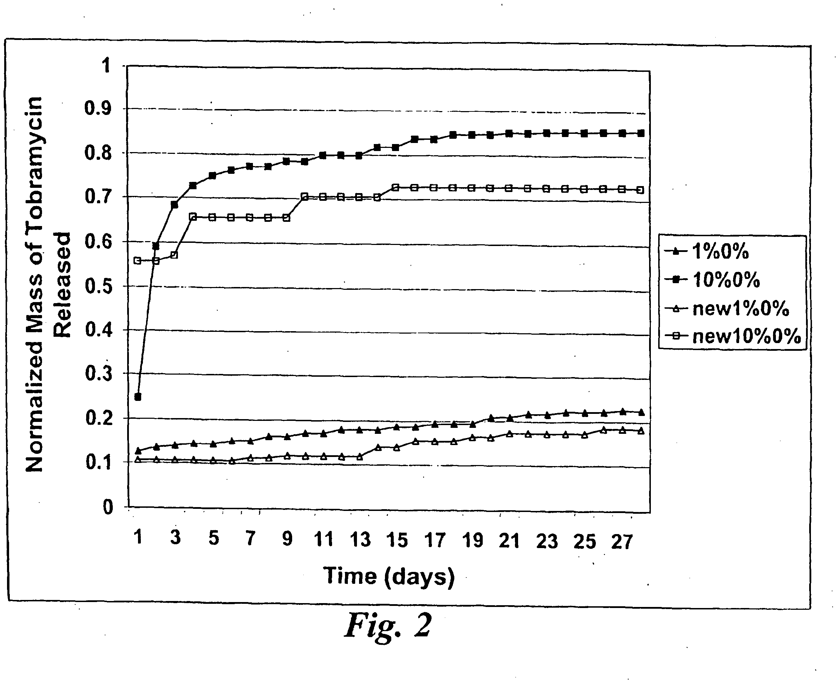

We performed a repeatability study where two of the formulations were manufactured more than one year apart. The *in vitro* elution rates for these experiments is presented in Fig. 2.

### In Vivo Results

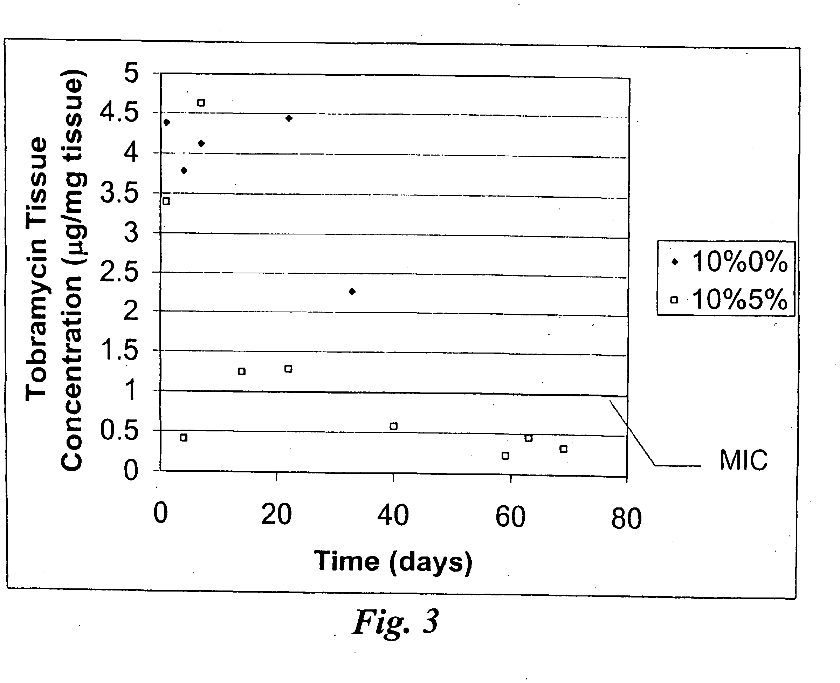

The *in-vivo* tobramycin concentrations are shown in Fig. 3 for the two formulations tested. The MIC of tobramycin against *S*. *Aureus* is shown for comparison. The histological scores for the quadriceps tissue is shown in Table 2 below.

**Table 2. Histological Scoring for Quadriceps Tissue**

| Timepoint | Histological Inflammation Score | |
|---|---|---|
| Days | 10% tobramycin | 10% tobramycin |
| | 0% PEG | 5% PEG |
| 1 | 1 | |
| 4 | 3 | |
| 7 | 3 | |
| 14 | | |
| 21 | 1 | 1 |
| 30-40 | 1 | |

The results of the *in-vitro* studies demonstrate that both changing the antibiotic concentration and the concentration of PEG can alter the elution characteristics of the antibiotic. In general, increasing the concentration of either component decreased the rate at which the antibiotic was released, although the initial burst of drug released increased with increasing antibiotic or PEG concentration. In all formulations the release rate leveled off to a near linear rate after the first week and remained steady for the next three weeks. At these linear release rates, it was determined that the formulation with 10% tobramycin and 0% PEG would have released all of the antibiotic in 60 days. By contrast, the formulation with 1% tobramycin and 0% PEG would take nearly 186 days to release all of the antibiotic. As can be seen from Fig. 2, we can reproducibly manufacture the different microsphere formulations.

In results of the *in-vivo* study demonstrate that these microspheres do not elicit an extreme inflammation response. The inflammation did increase to marked by day 3, but returned to minimal levels by day 7 and remained there for the next three weeks. This inflammation was localized to the implant site and did not produce visible signs of inflammation nor did it affect the animal's appetite or ambulation.

The most important result was that although the *in-vitro* elution characteristics demonstrated a larger linear release rate of tobramycin for the 10% tobramycin 5% PEG formulation, the *in-vivo* results showed higher tissue concentrations of tobramycin for the 10% tobramycin 0% PEG formulation, in fact, although the tissue levels were measurable for the 10% tobramycin 5% PEG formulation throughout the study, they remained at or below the minimum inhibitory concentration (MIC) for *S*. *aureus* in the second through fourth week. By contrast, the 10% tobramycin 0% PEG formulation resulted in tissue concentrations at least twice the MIC for the entire study period.

Microspheres were visible with the histological examination indicating that the microspheres do remain at the site of implantation for at least thirty days, and indeed we found measurable tobramycin levels in the tissue for both formulations of microspheres throughout the length of the study.

The results of this study suggest that microspheres made of PLGA and tobramycin, with or without PEG, make a suitable biodegradable drug delivery system. These microspheres do not elicit an undesirable inflammatory response, and the formulation can be adjusted to vary the release kinetics of the antibiotic. The microspheres deliver the antibiotic at a near-linear rate for at least four to six weeks. The microspheres remain at the site of implantation but are too small to inhibit tissue regeneration, a characteristic not shared by other suggested antibiotic delivery systems.

### EXAMPLE 2 - PLGA/tobramycin/PMMA - Parenteral Antibiotics

To test the effectiveness in eradicating an established case of osteomyelitis, a study was conducted using a rabbit model of osteomyelitis, wherein we tested the two methods of local antibiotic therapy - the microspheres and polymethyl methacrylate (PMMA) bone cement against parenteral antibiotics.

### Materials and Methods

Forty New Zealand White adult male rabbits, weighing 3-4 kg were selected for this study. Each rabbit underwent an initial surgery to inoculate the radius with bacteria in a well-established procedure. Four weeks later, each rabbit was returned to the operating room for irrigation and debridement surgery and a wound culture. At the time of the second surgery, each animal was randomly placed into one of 5 groups for treatment of the infection:
(1). *Control*: control group treated with PLGA microspheres containing no antibiotic,
(2). *Microspheres*: PLGA microspheres with 10% tobramycin,
(3). *Microspheres* + *Parenteral*: PLGA microspheres with 10% tobramycin and parenteral Ancef,
(4). *Cement* + *Parenteral*: PMMA bead with tobramycin and parenteral Ancef, and
(5). *Parenteral*: parenteral Ancef.

Each animal underwent treatment for four weeks before sacrifice. All animal procedures were approved by our institution's Animal Welfare Committee.

### Preparation of the PLGA Microspheres

The double emulsion solvent extraction technique, as described previously, was used to produce microspheres of approximately 15-20 µm in diameter containing approximately 10% by weight tobramycin (Nebcin®), from Eli Lilly, Indianapolis, IN. and 90% by weight 50:50 PLGA (Medisorb®), from Alkermes, Cincinnati, OH. These microspheres were blanketed with nitrogen gas, placed in closed vials, and stored frozen at -70° C until used. Two days prior to surgery the microspheres were sterilized using ethylene oxide gas. For each treated animal, 50mg of sterilized microspheres was implanted in the debrided bone defect.

### Preparation of the PMMA Beads

At the time of irrigation and debridement surgery, PMMA beads were prepared by mixing 20g of polymethyl methacrylate bone cement (Orthoset®), from Wright Medical, Arlington, TN, with 0.6g of tobramycin (Nebcin®). The resulting mixture was formed into beads of approximately 4mm diameter, weighing approximately 0.3g. One bead was placed into each debrided radius for treatment.

### Preparation of the S. aureus Inoculate

The strain of *S*. *aureus* used in this study, UAMS-1, was isolated from a patient with osteomyelitis and deposited at the American Type Culture Collection as strain ATCC 49230. The bacteria were prepared from overnight cultures grown in tryptic soy broth at 37°C with aeration. Cells were harvested by centrifugation, washed with sterile physiological saline, and resuspended to a final concentration of 2 x 10⁸ CFU/ml (OD of 60% transmittance). Cell suspensions were prepared on the day of surgery and held on ice until implanted.

Minimum inhibitory concentration (MIC) and minimum bactericidal concentration (MBC) for the two antibiotics tested, tobramycin and cefazolin, were determined by standard dilution methods published by the National Committee for Clinical Laboratory Standards. Briefly, *S*. *aureus* cells were grown and diluted to 0.5 McFarland turbidity standard, approximately 2 X 10⁸ cells/ml. The cells were mixed with either of the two antibiotics tested, at concentrations ranging from 2ug/ml to 64ug/ml. The following day, the cultures were examined for turbidity to allow determination of MIC values. After this, sample clear cultures were plated to determine the MBC, and colonies counts were done the next day.

### Surgical Procedure - Inoculation

All animals were fasted for 24 hours prior to surgery. Anesthesia was induced with ketamine (40mg/kg) and xylazine (0.5mg/kg) SQ injection. Anesthesia was maintained using isoflurane titrated to effect. The wound site was propped with betadine followed by a 70% ethanol rinse, and painted with Prepodyne prior to incision. The incision was made on anterior surface and extended down to the surface of the radius. The periosteum was sharply incised and elevated from the midshaft. A MicroHall oscillating saw was used to excise a 1cm segment from the midshaft of the radius. An inoculum of 10µl (2 x 10⁶ CPU) *S*. *aureus* was delivered by microinjection with a sterile pipette tip with an outside diameter of 0.56mm directly into the center of the medullary canal. The segment was replaced in its original position and the wound closed. All animals were monitored daily for 4 weeks for food and water intake, ambulatory status, and presence of localized and systemic infection (wound swelling, fever, etc.).

### Surgical Procedure - Irrigation and Debridement

Four weeks following the date of the initial surgery, the animals were fasted and prepared for the second surgery. Surgical preparation was exactly the same. Once the wound was opened, the infected bone was swabbed and the swab sent for culture. All infected soft tissues and infected bone were removed. The wound was irrigated with 40cc normal saline through a syringe. If treatment involved a local drug delivery system (groups 1-4), this system was placed before the wound was closed.

Post-operative care included administration of 25 mg/kg cefazolin SC BID (Bums Veterinary Supply, Farmers Branch, TX) for animals in groups 3, 4, and 5. For groups 2, 3 and 4, serum and urine were collected three times/day for the first day, once a day for days 2-7, three times/week for week 2, twice/week for weeks 3 and 4. The collected serum and urine samples were assayed for tobramycin concentration. All tobramycin concentrations were performed using fluorescence polarization immunoassay (Abbot TDx System). Sensitivity of the tobramycin assay is defined as the lowest measurable concentration which can be distinguished from zero with 95% confidence and was determined to be 0.18 microgram per milliliter.

### Sacrifice and Testing

All animals were sacrificed using an overdose of anesthesia (50-60mg/kg Pentobarbital administered IV). Weights were obtained. If serum had not been obtained in the week preceding sacrifice, it was obtained at the time of sacrificed and stored frozen until assayed. The radius was removed from each animal and AP and lateral X-rays were obtained. Each x-ray was labeled with tattoo number and the date. The radiographs were evaluated by two blinded observers according to the radiographic grading scale shown in Table 3, below.

**Table 3. Radiographic Grading Scale**

| Categories | | Scores |
|---|---|---|
| Size of Defect (length in mm at longest point) | | 0-10 |
| | New Bone Formation | |
| | Full (2 cortices + matrix) | 0 |
| | Moderate (2 cortices, no matrix) | 1 |
| | Mild (1 cortex) | 2 |
| | None | 3 |
| Maximum (worst) Score | | 13 |

The forelimb was then stripped of skin and soft tissues and cultures were obtained by swabbing the defect site with a culturette, which was sent for species identification.

Bone samples from the infected radius were divided so that both tobramycin assay and histology analysis could be performed. A 2cm piece of radius that surrounded the infection site was isolated using a Dremel saw. This section was divided into proximal and distal halves. One half was randomly chosen and pulverized after freezing in liquid nitrogen (MicroCryoCrusher®, BioSpec Products, Bartlesville, OK). The pulverized bone was placed into a glass vial of known weight, weighed and 0.5 cc of PBS was added. This sample was incubated in a 37°C water bath for 2 hours. The sample was then filtered into a cryogenic container and refrigerated at 4°C until the assay was performed. The remaining half was placed in a vial containing 10% NBF. Histological samples were decalcified, embedded in paraffin and sections were stained with H&E and Gram stains. These slides were evaluated by a pathologist according to the grading scale given in Table 4, below.

**Table 4. Histological Grading Scale**

| Categories | | Scores |
|---|---|---|
| Presence of Bacteria | | |
| | Marked | 3 |
| | Moderate | 2 |
| | Mild | 1 |
| | None | 0 |
| Intraosseous Inflammation | | |
| | Severe, abscess with fibrosis | 3 |
| | Moderate, with fibrosis | 2 |
| | Mild, with fibrosis | 1 |
| | None, fibrosis only | 0 |
| New Bone Formation | | |
| | Minimal - <25% | 3 |
| | Mild - 25-50% | 2 |
| | Moderate - 50-75% | 1 |
| Full - 75-100% | | 0 |
| | | |
| Maximum (worst) Score | | 9 |

### Results

Tables 5 and 6 show the minimum inhibitory concentration (MIC) and minimum bactericidal concentration (MBC) of tobramycin and cefazolin for this strain of *S*. *aureus* bacteria. The numbers are consistent with published values for strains of MRSA.

**Table. 5**

| Antibiotic | MIC (µg/ml) | MBC (µg/ml) |
|---|---|---|
| Cefazolin | 2 | 32 |
| Tobramycin | 4-8 | 16 |

**Table 6. MIC and MBC Determinations**

| Concentration of | Turbidity (MIC) | | Colonv # (MBC) | |
|---|---|---|---|---|
| Antibiotic (µg/ml) | T | C | T | C |
| 0 | ++ | ++ | ND | ND |
| 2 | + | - | ND | ND |
| 4 | + | - | ND | 125 |
| 8 | - | - | 120 | ND |
| 16 | - | - | 0 | 86 |
| 32 | - | - | 0 | 0 |
| 64 | - | - | 0 | 0 |

| | | | | |
|---|---|---|---|---|
| T= Tobramycin C= Cefazolin | | | | |

All rabbits became infected after the inoculum surgery; 100% of the cultures taken at the irrigation and debridement surgery were positive for *S*. *aureus.* Most animals developed signs of localized infection such as swelling or drainage at the surgical site but no animals showed signs of systemic disease. All animals were monitored daily for signs of discomfort and were treated to reduce discomfort. Supplemental food was given to animals with.diminished appetite and rubber mats were placed in cages to make ambulation more comfortable. After treatment with parenteral cefazolin, some animals had to be treated with metronidazole (Flagyl®, Bums Veterinary Supply) for diarrhea. Three animals died prematurely due to diarrhea.

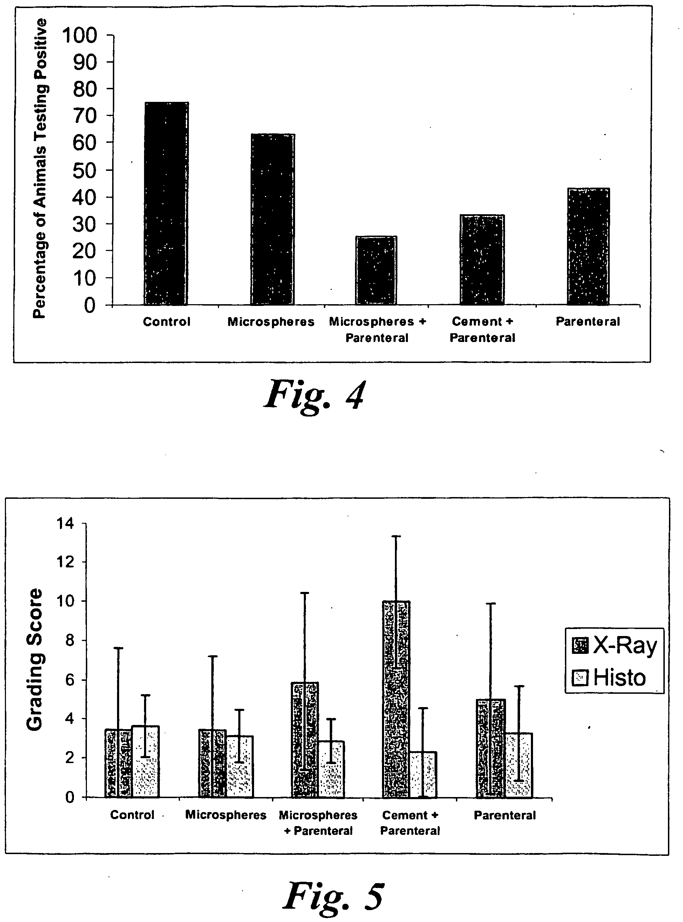

At sacrifice, the percentage of animals testing positive for osteomyelitis ranged from a maximum of 75% in the *Control* group (1) to a minimum of 25% in the *Microspheres* + *Parenteral* group (3) as shown in Fig. 4. Chi-square contingency table analysis shows that only the *Microspheres* + *Parenteral* group (3) had a significantly lower percentage than the *Control* group (1) (p=.046). However, if all of the groups where parenteral antibiotics were given are lumped together, and the *Control* and *Microspheres* groups (1) and (2) are lumped together, these are significantly different (p=.033).

Fig. 5 shows the results of the radiographic and histological grading of the specimens. In the radiographic grading scale, the *Cement* + *Parenteral* group (4) score significantly worse than the *Control* (1), *Microspheres* (2), and *Parenteral* (5) groups (p=.047). In the histological grading, none of the groups were significantly different.

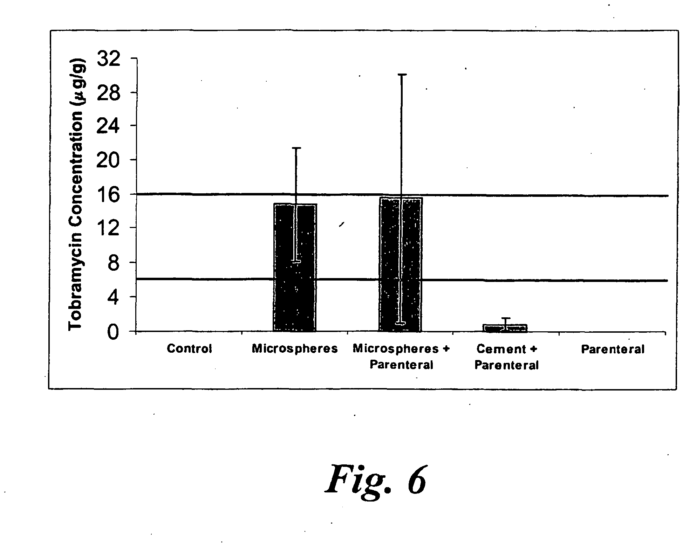

Fig. 6 shows the concentration of tobramycin in the bones for the groups treated locally with tobramycin. At four weeks after implantation of the local carrier system, the microspheres were still releasing significant amounts of tobramycin. The cement samples had small but measurable amounts of tobramycin. All but two of the microsphere samples had concentrations of tobramycin above the MIC and near the MBC level for the bacteria tested, whereas none of the PMMA samples reached the MIC level. None of the tested serum and urine specimens had measurable levels of tobramycin.

We have developed and described herein, tobramycin-loaded microspheres as a biodegradable drug delivery system for the treatment of osteomyelitis. These microspheres are spherical in shape with an average size of 20µm. The PLGA copolymers are biocompatible, biodegradable, and approved by the FDA for certain human clinical uses. *In-vitro* and *in-vivo* testing in muscle demonstrated that these microspheres deliver antibiotics for longer than four weeks and at nearly linear rates.

We have demonstrated the effectiveness of these microspheres in a rabbit model of osteomyelitis. In this study, all of the animals developed osteomyelitis by four weeks post inoculation. After the second surgery for irrigation and debridement of the wound, most of the animals showed signs of improvement. 25% of the animals in the *Control* group (1) showed no signs of infection at sacrifice. The only treatment group to demonstrate a significant improvement over the Control was the *Microspheres* + *Parenteral* group (3), where 75% percent of the animals showed no signs of infection at sacrifice. No treatment resulted in a 100% success rate.

Thus, the microspheres in accordance with the present invention resulted in high concentrations of tobramycin in the bone four weeks after implantation. The cement beads, by contrast, were still eluting tobramycin but at levels far below the MIC and MBC for the organism studied. In addition, the cement beads created a physical barrier against new bone formation in the debrided infection site. It was this phenomenon that resulted in the *Cement* + *Parenteral* group (4) having high (poorer) scores on the radiographic evaluation. Although the high bone tissue levels of tobramycin indicated that the microspheres remained at the site of implantation, the microspheres were small enough to allow new bone formation and degradation of the carrier (PLGA) occurred.

The histological scores indicated that there were no significant differences among any of the five groups studied. Thus, neither the microspheres nor the cement beads resulted in a chronic inflammatory response in the local tissues.

We have also demonstrated that these PLGA microspheres deliver antibiotic to the bone tissue at concentrations above or near the MBC for at least four weeks. At four weeks after the onset of treatment, the *Microspheres* + *parenteral* group (3) was the only group to demonstrate a significant improvement over the *Control* group (1). The microspheres in accordance with the present invention do not impede the formation of new bone growth into the debrided site, and do not require a second surgery for removal. The microspheres are biodegradable and do not result in chronic inflammation.

### EXAMPLE 3 - PLGA/vancomycin/PMMA Microspheres

We performed studies similar to the examples and formulations described above, with vancomycin substituted for tobramycin. In these experiments, we produced microspheres of about 6.86µm (microns) in diameter containing approximately 5% by weight vancomycin, and microspheres of about 7.46µm (microns) in diameter containing approximately 10% by weight vancomycin, and 90% by weight 50:50 PLGA. The percentage of PEG in the formulations was either 0% or 5%.

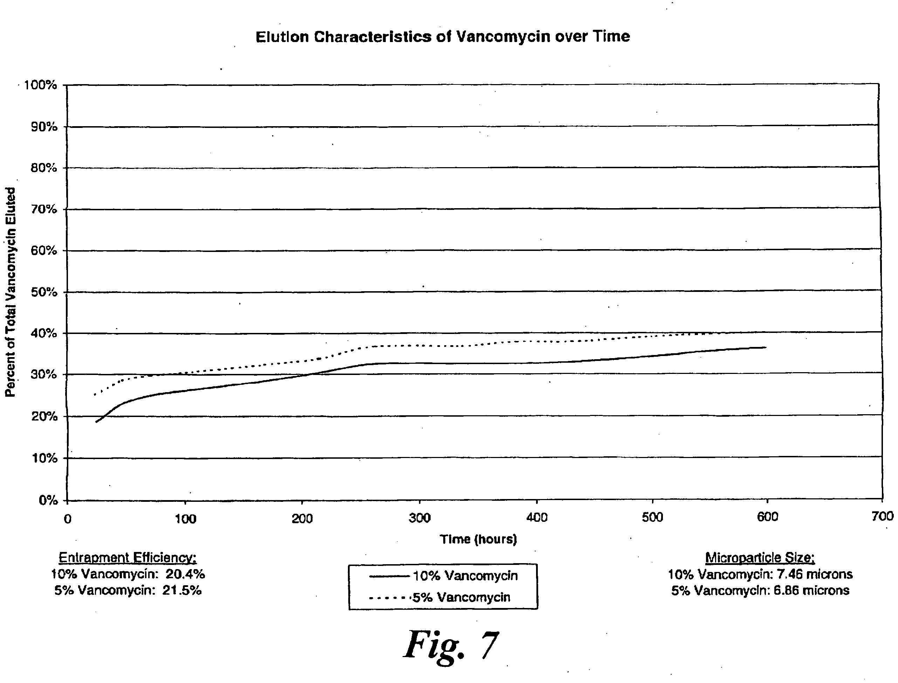

The results of the entrapment efficiencies and elution rates of vancomycin over a period of 600 hours are presented in Fig. 7. The result was that over a 600 hour period, in the 10% vancomycin formulation approximately 27% of the vancomycin was eluted, and in the 5% vancomycin formulation approximately 40% of the vancomycin was eluted. The entrapment efficiency of the 10% vancomycin formulation was found to be approximately 20.4% for the 10% vancomycin formulation and an entrapment efficiency of 21.5% for the 5% vancomycin formulation.

The vancomycin formulation, like the tobramycin formulation is eluted in a very good steady state manner. In both formulations, the levels were acceptable, with only slight differences in entrapment and release. Each of these formulations has advantages, for instance, one may be used as prophylaxis, while the other used for treatment of infection.

Although several antibiotics have been described herein for purposes of example, it should be understood the microspheres of the present invention may utilize various antibiotics and antibacterial agents or combinations thereof, preferably those in the class of "cephalosporins". These may be obtained commercially or be prepared according to the references cited in PHYSICIANS' DESK REFERENCE and the US ***FDA's Orange book*.**

For example, the present invention may utilize one or more of the following commercially available antibiotics and antibacterial agents selected from the group consisting of: Ancef, Tobramycin, Cefadroxil, Cefazolin, Cephalexin, Cefaclor, Cefotetan, Cefoxitin, Cefprozil, Cefuroxime, Loracarbef, Cefdinir, Cefixime, Cefoperazone, Cefotaxime, Cefpodoxime, Ceftazidime, Ceftibuten, Ceftozoxime, Ceftriaxone, Cefepime, and Vancomycin.

The present controlled release antibiotic microspheres may be implanted injected, or otherwise placed totally or partially within the body at a site of actual or potential infection and deliver an effective amount of the antibiotic agent sufficient to produce bactericidal levels in the body tissues and deliver a near-linear dosage of the antibiotic for at least 4 weeks at levels exceeding the minimum inhibitory concentration (MIC) for organisms commonly found to be the cause of the infections. The microspheres may be placed at a site of surgical treatment, such as a site of a bone fracture, at a site of placement of metal rods, plates or metallic fixators and joint replacement devices, or at a site of placement of graft materials used in cardiovascular, general, gynecologic, and neurosurgical procedures.

## Claims

1. Controlled release formulations capable of near-linear release of an antibiotic for a predetermined period of time for use in the treatment and prevention of infections involving a body at a site of actual or potential infection, comprising:
biodegradable microspheres formed of from 85% to 99% by weight of polylactic-co-glycolic acid (PLGA) in a ratio of 50% lactic to 50% glycolic acid;
from 0% to 5% by weight of polyethylene glycol (PEG); and
an aqueous antibiotic solution entrapped in said microspheres and capable of being eluted from said microspheres,
wherein said microspheres are from 6µm to 20µm in diameter whereby they are of a size sufficient not to impede the formation of new bone growth and to be capable of remaining at said site where the formation of new bone growth is desired, when said microspheres are placed at said site.

2. The controlled release formulations for use according to claim 1, wherein said antibiotic comprises from 1% to 10% by weight.

3. The controlled release formulations for use according to claim 1, wherein said antibiotic is selected from the class of cephalosporin antibiotics.

4. The controlled release formulations for use according to claim 1, wherein said antibiotic is selected from the group consisting of Ancef, Cefazolin, Tobramycin, and Vancomycin.

5. The controlled release formulations for use according to claim 1, wherein said antibiotic is selected from the group consisting of Ancef, Tobramycin, Cefadroxil, Cefazolin, Cephalexin, Cefactor, Cefotetan, Cefoxitin, Cefprozil, Cefuroxime, Loracarbef, Cefdinir, Cefixime, Cefoperazone, Cefotaxime, Cefpodoxime, Ceftazidizme, Ceftibuten, Ceftozoxime, Ceftriaxone, Cefepime, and Vancomycin.

6. The controlled release formulations for use according to claim 1, wherein said microspheres are from 15 µm to 20 µm in diameter

7. The use of controlled release as defined by any one of claims 1 to 6 in the manufacture of a medicament for treatment of infections involving the body at said site where the formation of new bone growth is desired.

8. The use according to claim 7 for administration at a site of surgical treatment.

9. The use according to claim 7 for administration at a site of a bone fracture.

10. The use according to claim 7 for administration at a site of placement of metal rods, plates or metallic fixators.

11. The use according to claim 7 for administration at a site of joint replacement devices.

12. The use according to claim 7 for placing at a site of osteomyelitis

13. A drug delivery system comprising a multiplicity of microspheres comprising an aqueous antibiotic solution encapsulated in a polymeric membrane comprising polyethylene glycol (PEG) and polylactic-co-glycolic acid (PLGA), wherein said PLGA is a 50:50 blend of lactic acid and glycolic acid, wherein said microspheres are from 6µm to 20µm in diameter, being of a size sufficient to not impede the formation of new bone growth and capable of remaining at a site of actual or potential infection in a body where the formation of new bone growth is desired, and wherein said encapsulated antibiotic solution is capable of controlled release.

## Patentansprüche

1. Formulierungen geregelter Freisetzung, die einer fast linearen Freisetzung eines Antibiotikums für eine vorbestimmte Zeitspanne fähig sind, zur Verwendung bei der Behandlung und Prävention von Infektionen, die einen Körper involvieren, an einer Stelle tatsächlicher oder potentieller Infektion, umfassend:
biologisch abbaubare Mikrokugeln, die aus 85 Gew.-% bis 99 Gew.-% Polymilch-co-glykolsäure (PLGA) in einem Gewichtsverhältnis von 50 % Milch- zu 50 % Glykolsäure gebildet sind;
0 Gew.-% bis 5 Gew.-% Polyethylenglykol (PEG); und
eine wässrige Antibiotikumlösung, die in den Mikrokugeln eingeschlossen und in der Lage ist, aus den Mikrokugeln eluiert zu werden,
wobei die Mikrokugeln einen Durchmesser von 6 µm bis 20 µm aufweisen, wobei sie eine Größe aufweisen, die ausreicht, die Bildung von neuem Knochenwachstum nicht zu behindern und in der Lage sind, an der Stelle zu bleiben, wo die Bildung von neuem Knochenwachstum erwünscht ist, wenn die Mikrokugeln an diese Stelle positioniert werden.

2. Formulierungen geregelter Freisetzung zur Verwendung nach Anspruch 1, wobei das Antibiotikum 1 Gew.-% bis 10 Gew.-% umfasst.

3. Formulierungen geregelter Freisetzung zur Verwendung nach Anspruch 1, wobei das Antibiotikum aus der Klasse von Cephalosporin-Antibiotika ausgewählt ist.

4. Formulierungen geregelter Freisetzung zur Verwendung nach Anspruch 1, wobei das Antibiotikum aus der Gruppe ausgewählt ist bestehend aus Ancef, Cefazolin, Tombramycin und Vancomycin.

5. Formulierungen geregelter Freisetzung zur Verwendung nach Anspruch 1, wobei das Antibiotikum aus der Gruppe ausgewählt ist bestehend aus Ancef Tombramycin, Cefadroxil, Cefalozin, Cephalexin, Cefaclor, Cefotetan, Cefoxitin, Cafprozil, Cefuroxim, Loracarbef, Cefdinir, Cefixim, Cefoperazon, Cefotaxim, Cefpodoxim, Ceftazidim, Ceftibuten, Ceftozoxim, Ceftriaxon, Cefepim und Vancomyzin.

6. Formulierungen geregelter Freisetzung zur Verwendung nach Anspruch 1, wobei die Mikrokugeln einen Durchmesser von 15 µm bis 20 µm aufweisen.

7. Verwendung von Formulierungen geregelter Freisetzung, wie in einem der Ansprüche 1 bis 6 definiert, bei der Herstellung eines Medikaments zur Behandlung von Infektionen, die den Körper involvieren, an einer Stelle, wo die Bildung von neuem Knochenwachstum erwünscht ist.

8. Verwendung nach Anspruch 7 zur Verabreichung an einer Stelle chirurgischer Behandlung.

9. Verwendung nach Anspruch 7 zur Verabreichung an einer Stelle eines Knochenbruchs.

10. Verwendung nach Anspruch 7 zur Verabreichung an einer Stelle des Positionierens von Metallstäben, -platten oder Metallfixateuren.

11. Verwendung nach Anspruch 7 zur Verabreichung an einer Stelle von Gelenkersatzvorrichtungen.

12. Verwendung nach Anspruch 7 zum Positionieren an einer Stelle von Osteomyelitis.

13. Arzneimittelabgabesystem umfassend eine Mehrzahl von Mikrokugeln umfassend eine wässrige Antibiotikumlösung, die in einer polymeren Membran eingekapselt ist, die Polyethylenglykol (PEG) und Polymilch-co-glykolsäure (PLGA) umfasst, wobei die PLGA eine Mischung von 50:50 Milchsäure und Glykolsäure ist, wobei die Mikrokugeln einen Durchmesser von 6 µm bis 20 µm aufweisen, wobei sie eine Größe aufweisen, die ausreicht, die Bildung von neuem Knochenwachstum nicht zu behindern und in der Lage sind, an einer Stelle tatsächlicher oder potentieller Infektion in einem Körper zu bleiben, wo die Bildung von neuem Knochenwachstum erwünscht ist, und wobei die eingekapselte Antibiotikumlösung der kontrollierten Freisetzung fähig ist.

## Revendications

1. Formulations à libération régulée capable d'une libération proche du linéaire d'un antibiotique pendant une période de temps prédéterminée pour une utilisation dans le traitement et la prévention d'infections impliquant un corps à un site d'infection réelle ou potentielle, comprenant:
des microsphères biodégradables formées de 85% à 99% en poids d'acide polylactique-co-glycolique (PLGA) dans un rapport de 50% d'acide lactique sur 50% d'acide glycolique;
de 0% à 5% en poids de polyéthylèneglycol (PEG); et
une solution aqueuse d'antibiotique piégée dans lesdites microsphères et capable d'être éluée à partir desdites microsphères,
dans lesquelles lesdites microspheres sont de 6 µm à 20 µm de diamètre, en conséquence de quoi elles sont d'une taille suffisante pour ne pas entraver la formation de croissance d'os nouveau et pour être capables de rester au niveau dudit site où la formation de croissance d'os nouveau est désirée, lorsque lesdites microsphères sont placées au niveau dudit site.

2. Formulations à libération régulée pour une utilisation selon la revendication 1, dans lesquelles ledit antibiotique comprend de 1% à 10% en poids.

3. Formulations à libération régulée pour une utilisation selon la revendication 1, dans lesquelles ledit antibiotique est choisi dans la classe d'antibiotiques de céphalosporine.

4. Formulations à libération régulée pour une utilisation selon la revendication 1, dans lesquelles ledit antibiotique est choisi dans le groupe constitué d'ancef, de céphazoline, de tobramycine et de vancomycine.

5. Formulations à libération régulée pour une utilisation selon la revendication 1, dans lesquelles ledit antibiotique est choisi dans le groupe constitué d'ancef, de tobramycine, de céfadroxil, de céphazoline, de céphalexine, de céfactor, de céfotétan, de céfoxitine, de cefprozil, de céfuroxime, de loracarbef, de cefdinir, de céfixime, de céfopérazone, de céfotaxime, de cefpodoxime, de ceftazidime, de ceftibutène, de ceftozoxime, de ceftriaxone, de céfépime et de vancomycine.

6. Formulations à libération régulée pour une utilisation selon la revendication 1, dans lesquelles lesdites microsphères sont de 15 µm à 20 µm de diamètre.

7. Utilisation de formulations à libération régulée selon l'une quelconque des revendications 1 à 6 dans la fabrication d'un médicament pour le traitement d'infections impliquant le corps au niveau dudit site où la formation de croissance d'os nouveau est désirée.

8. Utilisation selon la revendication 7, pour une administration à un site de traitement chirurgical.

9. Utilisation selon la revendication 7, pour une administration à un site d'une fracture osseuse.

10. Utilisation selon la revendication 7, pour une administration à un site de placement de tiges, de plaques de métal ou de dispositifs de fixation métalliques.

11. Utilisation selon la revendication 7, pour une administration à un site de dispositifs de remplacement d'articulation.

12. Utilisation selon la revendication 7, pour un placement à un site d'ostéomyélite.

13. Système de libération de médicament comprenant une multiplicité de microsphères comprenant une solution aqueuse d'antibiotique encapsulée dans une membrane polymère comprenant du polyéthylèneglycol (PEG) et de l'acide polylactique-co-glycolique (PLGA), dans lequel ledit PLGA est un mélange 50:50 d'acide lactique et d'acide glycolique, dans lequel lesdites microsphères sont de 6 µm à 20 µm de diamètre, qui sont d'une taille suffisante pour ne pas entraver la formation de croissance d'os nouveau et être capables de rester à un site d'infection réelle ou potentielle dans un corps où la formation de croissance d'os nouveau est désirée, et dans lequel ladite solution d'antibiotique encapsulée est capable d'une libération régulée.
